# EUROPEAN PATENT SPECIFICATION

(11) **EP 0 831 893 B1**
(45) Date of publication and mention of the grant of the patent: **07.05.2003**
(21) Application number: 96919130.3
(22) Date of filing: 05.06.1996
(51) Int. Cl.: A61K 38/57, A61P 29/00

(54) **REGULATION OF NEUTROPHIL ELASTASE SYNTHESIS AND RELEASE**
REGULIERUNG DER NEUTROPHIL ELASTASE SYNTHESE UND FREISETZUNG
REGULATION DE LA SYNTHESE ET DE LA LIBERATION DE LA NEUTROPHILE ELASTASE

(30) Priority: 07.06.1995 US 486384; 07.06.1995 US 483459
(43) Date of publication of application: 01.04.1998
(73) Proprietor: CHIRON CORPORATION, Emeryville, California 94608 (US)
(72) Inventor: JOHNSON, Kirk, Moraga, CA 94556 (US); CREASEY, Alba, A., Peidmont, CA 94611 (US); AARDEN, Lucien, A., NL-Amsterdam (NL)
(74) Representative: Goldin, Douglas Michael
(86) International application number: US9609154
(87) International publication number: WO96040224

(56) References cited:
- WO-A-93/14122
- WO-A-93/24143
- WO-A-95/18830
- WO-A-96/06637
- CIRCULATORY SHOCK 44 (3). 1994. 126-137, XP000602149 CARR C ET AL: "Recombinant E. coli-derived tissue factor pathway inhibitor reduces coagulopathic and lethal effects in the baboon gram-negative model of septic shock."
- THROMB HAEMOSTASIS 67 (5). 1992. 537-541, XP000602163 PETERSEN L C ET AL: "EFFECT OF LEUKOCYTE PROTEINASES ON TISSUE FACTOR PATHWAY INHIBITOR."
- 37TH ANNUAL MEETING OF THE AMERICAN SOCIETY OF HEMATOLOGY, SEATTLE, WASHINGTON, USA, DECEMBER 1-5, 1995. BLOOD 86 (10 SUPPL. 1). 1995. 875A, XP002013723 JOHNSON K ET AL: "The cytokine response to coagulation and endotoxin in whole blood: Regulation by the tissue factor pathway inhibitor."

## Description

### Technical Field

The invention relates to the use of Tissue Factor Pathway Inhibitor (IFPI) in the manufacture of a medicament to inhibit the synthesis and release of neutrophil elastase, and IL-8 and inhibit the activity of plasmin. It also relates to en in vitro method of measuing a pution respouse to TFPI.

### Background of the Invention

Tissue Factor Pathway Inhibitor (TFPI) inhibits the coagulation cascade in at least two ways: preventing formation of factor VIIa/tissue factor complex and by binding to the active site of factor Xa. The primary sequence of TFPI, deduced from cDNA sequence, indicates that the protein contains three Kunitz-type enzyme inhibitor domains. The first of these domains is required for the inhibition of the factor VIIa/tissue factor complex. The second Kunitz-type domain is needed for the inhibition of factor Xa. The function of the third Kunitz-type domain is unknown. TFPI has no known enzymatic activity and is thought to inhibit its protease targets in a stoichiometric manner; namely, binding of one TFPI Kunitz-type domain to the active site of one protease molecule. The carboxy-terminal end of TFPI is believed to have a role in cell surface localization via heparin binding and by interaction with phospholipid. TFPI is also known as Lipoprotein Associated Coagulation Inhibitor (LACI), Tissue Factor Inhibitor (TFI), and Extrinsic Pathway Inhibitor (EPI).

Mature TFPI is 276 amino acids in length with a negatively charged amino terminal end and a positively charged carboxy-terminal end. TFPI contains 18 cysteine residues and forms 9 disulphide bridges when correctly folded. The primary sequence also contains three Asn-X-Ser/Thr N-linked glycosylation consensus sites, the asparagine residues located at positions 145, 195 and 256. The carbohydrate component of mature TFPI is approximately 30% of the mass of the protein. However, data from proteolytic mapping and mass spectral data imply that the carbohydrate moieties are heterogeneous. TFPI is also found to be phosphorylated at the serine residue in position 2 of the protein to varying degrees. The phosphorylation does not appear to affect TFPI function.

TFPI has been isolated from human plasma and from human tissue culture cells including HepG2, Chang liver and SK hepatoma cells. Recombinant TFPI has been expressed in mouse C127 cells, baby hamster kidney cells, Chinese hamster ovary cells and human SK hepatoma cells. Recombinant TFPI from the mouse C127 cells has been shown in animal models to inhibit tissue-factor induced coagulation.

A non-glycosylated form of recombinant TFPI has been produced and isolated from *Escherichia coli* (*E. coli*) cells as disclosed in U.S. Pat. No. 5,212,091. This form of TFPI has been shown to be active in the inhibition of bovine factor Xa and in the inhibition of human tissue factor-induced coagulation in plasma. Methods have also been disclosed for purification of TFPI from yeast cell culture medium, such as in Petersen *et al*, J.Biol.Chem. 18:13344-13351 (1993).

Recently, another protein with a high degree of structural identity to TFPI has been identified. Sprecher *et al*, Proc. Nat. Acad. Sci., USA 91:3353-3357 (1994). The predicted secondary structure of this protein, called TFPI-2, is virtually identical to TFPI with 3 Kunitz-type domains, 9 cysteine-cysteine linkages, an acidic amino terminus and a basic carboxy-terminal tail. The three Kunitz-type domains of TFPI-2 exhibit 43%, 35% and 53% primary sequence identity with TFPI Kunitz-type domains 1, 2, and 3, respectively. Recombinant TFPI-2 strongly inhibits the amidolytic activity of factor VIIa/tissue factor. By contrast, TFPI-2 is a weak inhibitor of factor Xa amidolytic activity.

TFPI has been shown to prevent mortality in a lethal *Escherichia coli* (*E.coli*) septic shock baboon model. Creasey *et al*., J. Clin. Invest. 91:2850-2860 (1993). Administration of TFPI at 6 mg/kg body weight shortly after infusion of a lethal dose of *E. coli* resulted in survival in all five TFPI-treated animals with significant improvement in quality of life compared with a mean survival time for the five control animals of 39.9 hours. The administration of TFPI also resulted in significant attenuation of the coagulation response, of various measures of cell injury and significant reduction in pathology normally observed in *E.coli* sepsis target organs, including kidneys, adrenal glands, and lungs.

Due to its clot-inhibiting properties, TFPI may also be used to prevent thrombosis during microvascular surgery. For example, U.S. 5,276,015 discloses the use of TFPI in a method for reducing thrombogenicity of microvascular anastomoses wherein TFPI is administered at the site of the microvascular anastomoses contemporaneously with microvascular reconstruction.

WO 93/24143 discloses the use of LACI/TFPI to treat disease states associated with acute or chronic inflammation including sepsis and septic shock.

WO 93/14122 discloses variants of human Kunitz-type protease inhibitor domain I of TFPI for use in therapeutic applications suggested for native aprotinin or aprotinin analogues. (See also WO 93/14121 and WO 93/120 which disclose variants of Kunitz-type protease domain II and III respectively).

WO 95/18830 discloses variants of the first Kunitz domain of LACI/TFPI which inhibit plasmin. It also discloses the use of such mutants to prevent or treat disease states involving plasmin.

Neutrophil elastase release is linked to acute inflammatory diseases including ARDS and multiple organ failure. Idle *et al*., (1985) Am. Rev. Respire. Ids. 132:1098. Joshua, M., *et al*., (1994) Am. J. Respire. Crate. Care Med. 150:S123. Acute inflammatory reactions, including ARDS, reperfusion injury (including lung reperfusion injury), arthritis, and sepsis are also associated with the production of cytokines such as IL-8. IL-8 is thought to play an important role in the recruitment and activation of PMNs at inflammatory sites.

Currently, there is no single agent which might effectively inhibit both thrombosis due to activation of the extrinsic pathway of coagulation and the release of inflammatory mediators such as neutrophil elastase.

### Summary of the Invention

It has now been found that coagulation activation and LPS (the active moiety of bacterial endotoxin) synergize for elastase release and that TFPI inhibits elastase release induced by coagulation activation and by coagulation in the presence of LPS. Further, TFPI has been shown to inhibit plasmin activity at therapeutically relevant doses. Therefore, TFPI has been shown to be relevant and is useful in disease states involving inflammation resulting from elastase release. Accordingly, TFPI may be used for the manufacture of a medicament to treat such clinical indications as severe acute pancreatitis, emphysema, multiple organ failure and cystic fibrosis.

It has also been found that coagulation activation/clotting induces IL-8 production in normal human whole blood cultures. Moreover, it has been found that coagulation activation/clotting and LPS together in whole blood cultures synergize for increased IL-8 production. TFPI is able to block the IL-8 production induced under both circumstances.

Finally, the observations that TFPI inhibits the activity of plasmin and the synthesis and release of neutrophil elastase and IL-8 allow the use of assays for plasmin activity, and for elastase and IL-8 to be used to determine the patient's response to TFPI.

In one embodiment therefore the invention provides use of an agent in the manufacture of a medicament for treating a disease associated with increased synthesis and release of neutrophil elastase, said agent being capable of inhibiting (1) coagulation and (2) release of neutrophil elastase, wherein said agent is Tissue Factor Pathway Inhibitor (TFPI) or ala-TFPI, whereby one or more symptoms associated with said disease is reduced, and wherein the disease is selected from:
(a) severe acute pancreatitis;
(b) emphysema;
(c) multiple organ failure; and
(d) cystic fibrosis.

In a further embodiment the invention provides use of an agent in the manufacture of a medicament for preventing a symptom of a disease associated with increased synthesis and release of neutrophil elastase, said agent being capable of inhibiting (1) coagulation and (2) release of neutrophil elastase, wherein said agent is Tissue Factor Pathway Inhibitor (TFPI) or ala-TFPI, whereby the risk of developing said symptom in the individual is reduced, and wherein the disease is selected from:
(a) severe acute pancreatitis;
(b) emphysema; and
(c) cystic fibrosis.

The invention also provides an *in vitro* method of measuring a patient's response to TFPI, comprising; determining the level of one or more indicators of Tissue Factor Pathway Inhibitor (TFPI) efficacy in a sample removed from the patient, said one or more indicators being selected from neutrophil elastase; IL-8; and plasmin, wherein a decreased level of said one or more indicators indicates a response of the patient to TFPI.

In a preferred embodiment the patient whose response is being measured in the *in vitro* method, has been diagnosed with one or more diseases associated with increased synthesis and release of neutrophil elastase selected from:
(a) severe acute pancreatitis;
(b) emphysema;
(c) rheumatoid arthritis;
(d) multiple organ failure;
(e) cystic fibrosis;
(f) sepsis; and
(g) Adult Respiratory Distress Syndrome.

In another preferred embodiment, the patient whose response is being measured in the *in vitro* method has been diagnosed with one or more diseases associated with increased synthesis and release of IL-8 selected from:
(a) Adult Respiratory Distress Syndrome;
(b) reperfusion injury;
(c) sepsis; and
(d) arthritis.

### Brief Description of the Drawings

Figure 1 shows production of neutrophil elastase in undiluted whole blood cultures under the following conditions: control (clot); TFPI (10 µg/ml); LPS (1ng/ml)(clot); TFPI + LPS and heparin (50 u/ml).
Figure 2 shows production of neutrophil elastase in coagulating 1:10 whole blood culture containing various concentrations of hirudin.
Figure 3 shows production of neutrophil elastase in diluted (1:10) whole blood cultures containing various concentrations of TFPI and hirudin or heparin.
Figure 4 shows production of neutrophil elastase in diluted (1:10) whole blood cultures containing 1ng/ml LPS in addition to various concentrations of TFPI and hirudin or heparin.
Figure 5 displays the results of experiments showing that TFPI inhibits plasmin activity.
Figure 6 displays the results of experiments showing that IL-8 levels in whole blood culture are drastically reduced in the presence of TFPI.
Figure 7 shows the synergistic effect of coagulation activation/clotting and LPS in whole blood cultures on IL-8 levels.
Figures 8 and 9 show the results of time course experiments measuring IL-8 levels in whole blood cultures in the absence (Figure 8) and the presence (Figure 9) of LPS.
Figure 10 shows the effect of TFPI on cytokine production in whole blood cultures containing 5 U/ml Hirudin and 1 ng/ml LPS.

### Detailed Description of the Invention

### A. Definitions

As used herein, "TFPI" refers to mature Tissue Factor Pathway Inhibitor. As noted above, TFPI is also known in the art as Lipoprotein Associated Coagulation Inhibitor (LACI), Extrinsic Pathway Inhibitor (EPI) and Tissue Factor Inhibitor (or TFI).

TFPI which has been slightly modified for production in bacterial cells is encompassed in the definition. For example, a TFPI analog have an alanine residue at the amino-terminal end of the TFPI polypeptide has been produced in *Escherichia coli.* See U.S. 5,212,091.

As used herein, "pharmaceutically acceptable composition" refers to a composition that does not negate or reduce the biological activity of formulated TFPI, and that does not have any adverse biological effects when formulated TFPI is administered to a patient.

As used herein, "patient" encompasses human and veterinary patients.

### B. General Methods

TFPI may be prepared by recombinant methods as disclosed in U.S. 5,212,091.

Briefly, TFPI is expressed in *Escherichia coli* cells and the inclusion bodies containing TFPI are isolated from the rest of the cellular material. The inclusion bodies are subjected to sulfitolysis, purified using ion exchange chromatography, refolded by disulfide interchange reaction and the refolded, active TFPI purified by cation exchange chromatography. TFPI may also be produced in yeast.

### Whole Blood Culture

The whole blood culture system can be carried out as follows. Blood is collected from normal donors into anticoagulant. Venous blood from normal health donors was collected directly into clinical heparin or EDTA (K3) vacutainers (Baxter). Alternatively, venous blood was collected into sterile polypropylene syringes and immediately transferred into microtiter wells containing indicated concentrations of various additives including:

| | |
|---|---|
| a. | 20-50U/ml heparin (blood fully anticoagulated, even with 10X dilution) |
| b. | 50-60 U/ml hirudin (recombinant yeast, American Diagnostica) |
| c. | 10 µg/ml TFPI |
| d. | 1 U/ml heparin (ESI) |
| e. | 10 mM EDTA (for isolated neutrophils) |
| f. | 1 ng/ml LPS (*E. coli* Rc) (Sigma, St. Louis, MO) |
| g. | 3.8% citrate (for isolated PBMC). |

TFPI was formulated at 11mg/ml in 2M urea, 20mM sodium phosphate pH 7.2 and 0.14M NaCl. Blood collected into vacutainers was quickly transferred into polypropylene tubes prior to addition into culture wells.

Whole blood was cultured in 96 well microtiter plates (Corning) at a volume of 200 µl per well at 37C, 5% CO₂ for 2-48 hours in a humidified atmosphere. The blood will typically be at a final dilution of 1:8 to 1:10 in RPMI 1640 medium + 0.1% "low-endotoxin" fetal calf serum (FCS) (Hyclone, Logan, UT). The cultures were then spun down at 400 x g for 1 minute at 4°C. Supernatant liquids were then removed at various time points (typically 2-2.5 hours) for analysis of soluble mediators. In the event that clotting occurred during culturing, the contents of clotted wells and comparative groups were transferred to polypropylene microfuge tubes and briefly spun to pellet cells and fibrin clot prior to harvesting supernatants. Soluble mediators were measured in supernatants by ELISA or other bioassay.

### Peripheral Blood Mononuclear Cell (PBMC) Cultures

Whole blood was collected into EDTA vacutainers was layered over a ficoll gradient (NIM medium, Cardinal Assoc.) at a mixture of 7-8 ml blood onto 5 ml NIM medium in 15 ml polystyrene tubes. The tubes were spun at 500 x g for 30 minutes and the mononuclear cell layer was isolated as the top band in the gradient. In some experiments, PBMC were also isolated using citrated Cell Preparation Tubes (Becton-Dickinson, Mountain View, CA) wherein blood is collected and fractionated in the same tube. Identical results were obtain utilizing PBMC isolated in either manner. Following a sterile saline wash, PBMC were cultured at ∼1 x 10⁵ cells per well in RPMI/0.1% FCS as described above for whole blood cell cultures.

### Assay for Soluble Mediators

ELISA assays for elastase, IL-8, IL-6 and TNF were conducted as follows. 96 well microtiter plates were coated overnight with the appropriate antibodies. Plates were washed and samples were added to each well along with biotin-labelled antibody and serum. The plates were then incubated and washed. Streptavidin-horseradish peroxidase was then added to the wells and allowed to incubate. The wells were again washed and developed with TMB, sodium acetate and peroxide. The reaction was stopped by the addition of 2M sulfuric acid and plates read at O.D. 450 nm. When assaying for elastase, there is an incubation period between the addition of the samples and the biotinylated anti-elastase antibodies. For TNF, poly-streptavidin-horseradish peroxidase and milk are used instead of Strep-HRP and serum.

Spectrozyme Plasmin assay kits were obtained from American Diagnostica. Quantikine IL-1β ELISA kits were purchased from R&D Systems. The manufacturers' instructions were followed in completing the assays. Coagulation Activation

Measurement of the extent of coagulation activation was performed in a qualitative manner by observation of clotting and, quantitatively, via immunodetection of thrombin:antithrombin (TAT) complex and fibrinopeptide A levels according to manufacturer's protocols (Diagnostica Stago, France). Supernatants were also routinely analyzed for chromogenic activity against various substrates including Spectrozyme Xa and TH (thrombin) (American Diagnostica) for correlation with the above metrics as well as to confirm activity of purified factors added to isolated PBMC cultures including prothrombin, α-thrombin, and factor Xa.

### C. Examples

### Example 1

A culture system utilizing normal human blood wherein coagulation activation and clotting could be controlled and inflammatory mediator responses could be evaluated in the presence or absence of LPS was established. Essentially, blood is collected in concentrations of anticoagulant, such as the irreversible thrombin inhibitor hirudin. When cultured at final blood dilutions of 1:10, coagulation activation and clotting could be observed. The extent of coagulation activation and clotting can be controlled by appropriate additions of anticoagulants upon blood dilution.

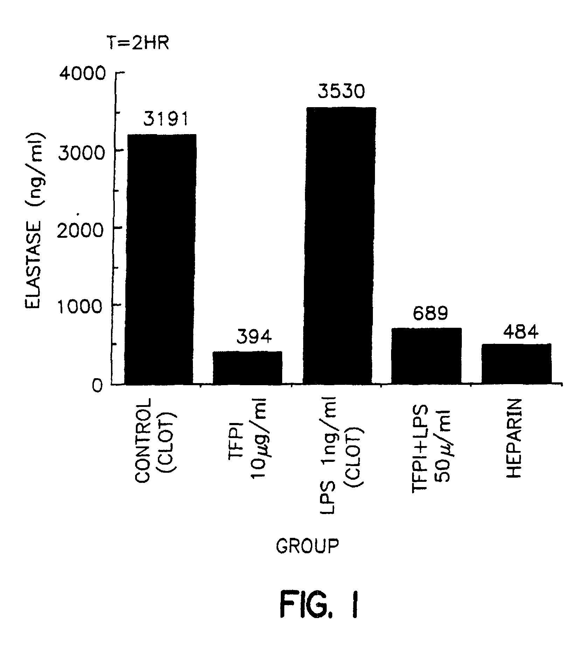

While most examinations of the effect of coagulation activation/clotting on elastase release have been performed in 1:10 diluted blood, the phenomena was observed in undiluted whole blood as indicated in Figure 1. The incubation was carried out for two hours. Clotting the whole blood resulted in significant elastase release in the supernatant as compared to blood treated with 50 U/ml heparin. In undiluted whole blood, the addition of LPS resulted in only a small increment in elastase production, probably because the coagulation signal itself is so strong in the culture. TFPI addition at t=0 abrogates the coagulation and coagulation+LPS induced elastase release.

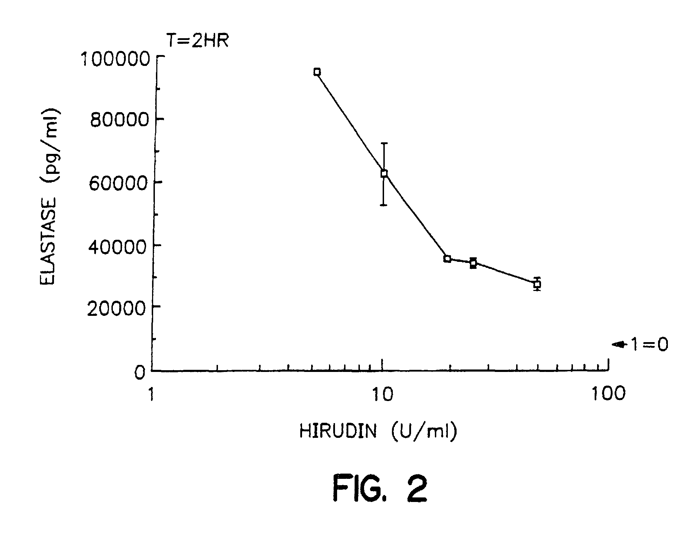

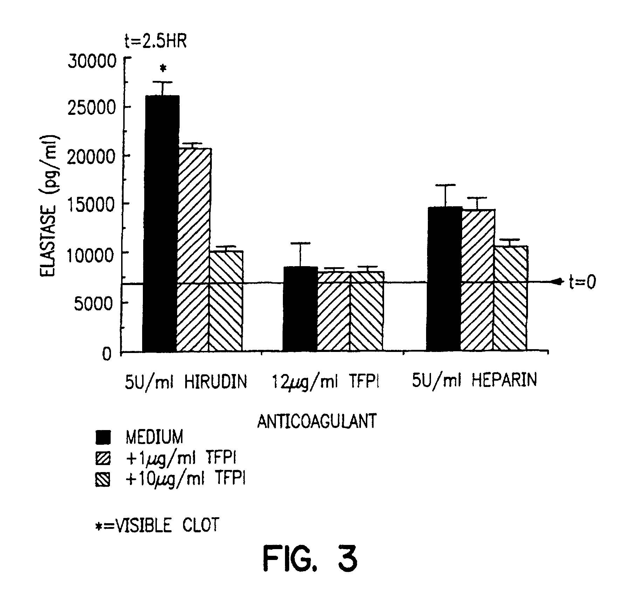

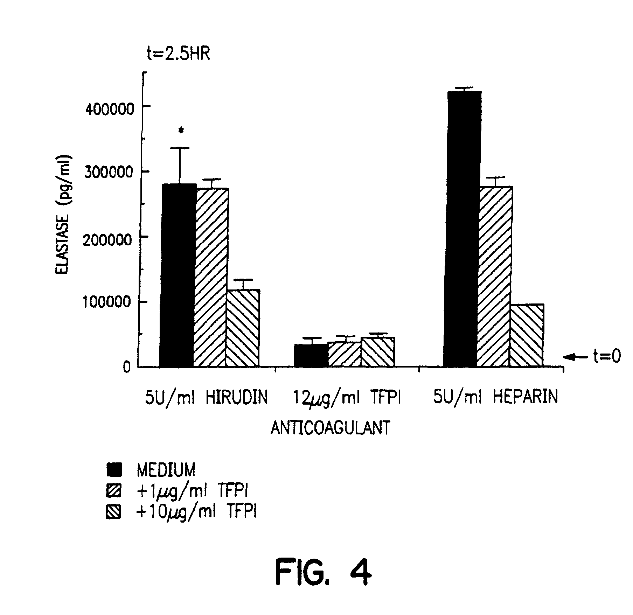

As shown in Figure 2, dilution of hirudin such that coagulation activation/clotting through thrombin can proceed is accompanied by elastase release which is most marked at low hirudin concentrations (5 & 10 U/ml) wherein clotting can be observed the time of harvest (t=2 hours). The addition of low concentrations of TFPI to hirudin-treated blood cultures (5 U/ml hirudin) blocks elastase release in a dose-dependent manner (Figure 3). Addition of LPS in the low-hirudin culture results in significantly more elastase production (Figure 4). Nonetheless, TFPI markedly inhibits elastase release.

In contrast to anticoagulated blood collected in heparin, blood collected into TFPI fails to exhibit the synergistic elastase release observed upon culture with LPS. Moreover, the coagulation/LPS-induced elastase release in heparin-anticoagulated blood can be inhibited by the addition of TFPI to the culture at t=0 (Figure 4).

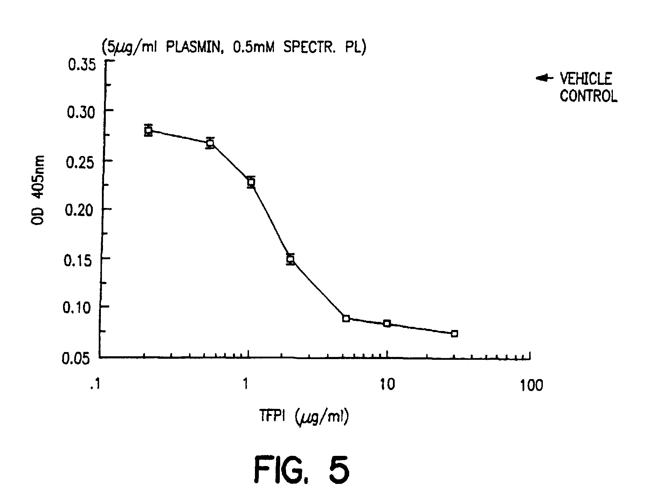

Finally, the effect of the presence of TFPI in the cultures on synthesis and release of plasmin was determined. Figure 5 shows that increasing concentrations of TFPI result in decreased detection of plasmin activity in the cultures. The inhibitory effect of TFPI on plasmin activity may therefore serve as a marker for efficacy of TFPI in patients.

### Example 2

Using the culture system described above, it has been found that IL-8 production increases as a result of the coagulation activation/clotting. Also, coagulation activation/clotting and LPS appear to have a synergistic effect for IL-8 synthesis and release.

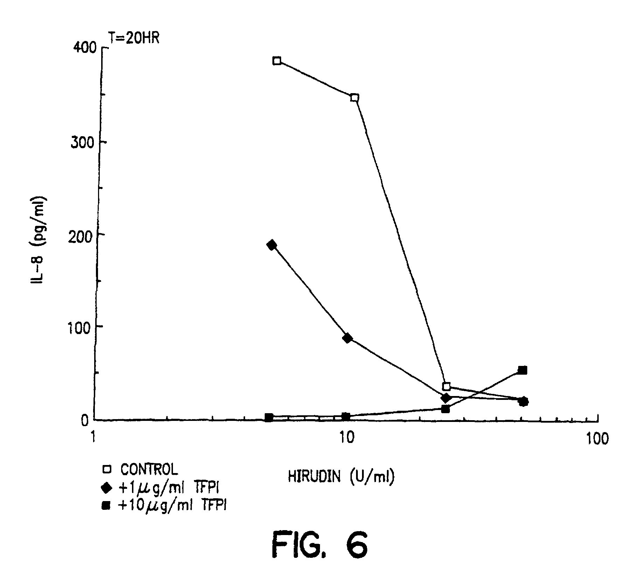

Dilution of hirudin in the blood cultures allows thrombin amplification of the coagulation cascade resulting in significant coagulation activation and observable clotting. Coincident with coagulation activation/clotting is the production of IL-8 into culture supernatants detectable by ELISA (Figure 6). TFPI inhibits the coagulation activation/clotting-induce IL-8 production in a dose dependent manner (Figure 6).

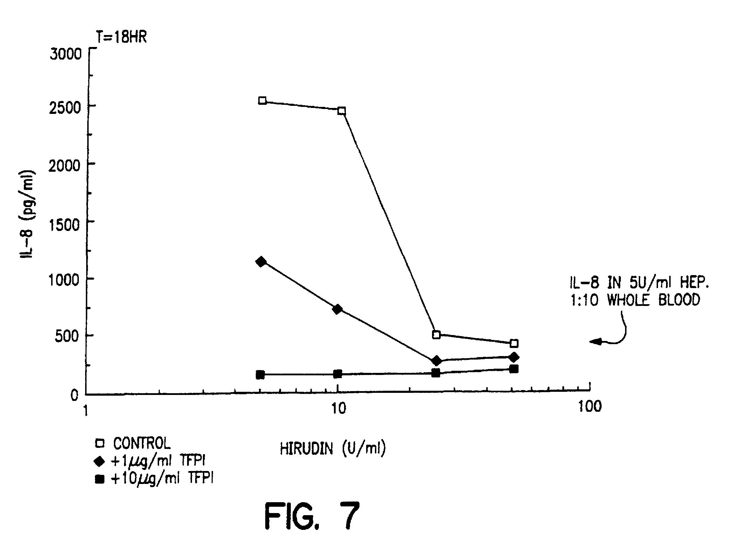

When LPS (1 ng/ml) is included in the hirudin-treated blood cultures, a synergistic increase in IL-8 production is observed under conditions where significant coagulation activation/clotting occurs (i.e. 5 - 10 U/ml hirudin) (Figure 7). The response is synergistic because coagulation activation/clotting results in ∼350pg/ml IL-8 and LPS induces ∼450 pg/ml IL-8 under fully anticoagulated conditions (50 U/ml hirudin or 5 U/ml heparin), but the combinations of coagulation activation/clotting and LPS results in ∼2500 pg/ml IL-8 production. As shown in Figure 7, TFPI inhibits the synergistic IL-8 production in a dose-dependent manner.

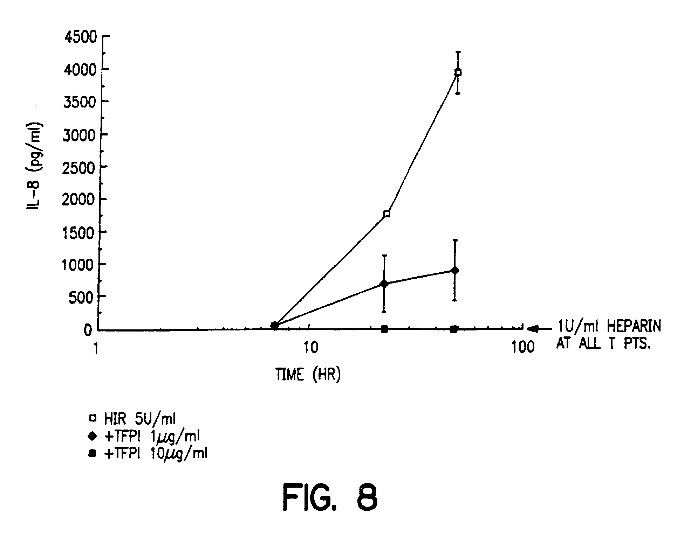

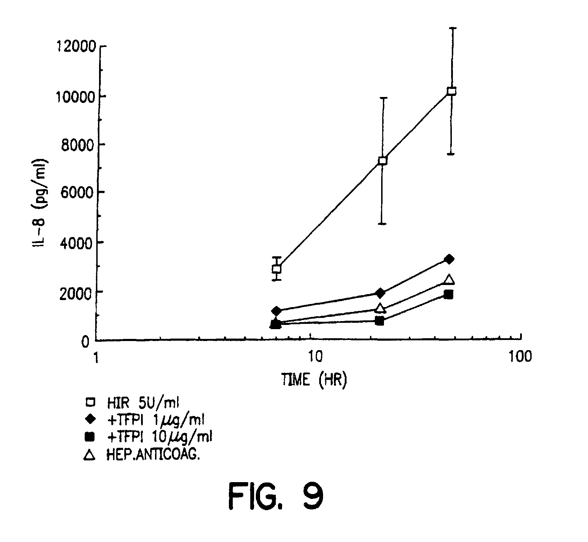

The ability of TFPI to abrogate IL-8 production induced by coagulation activation/clotting or the combination of coagulation activation/clotting + LPS is not due to altered kinetics of cytokine production (Figures 8 and 9). TFPI inhibits induced IL-8 production at all time points evaluated.

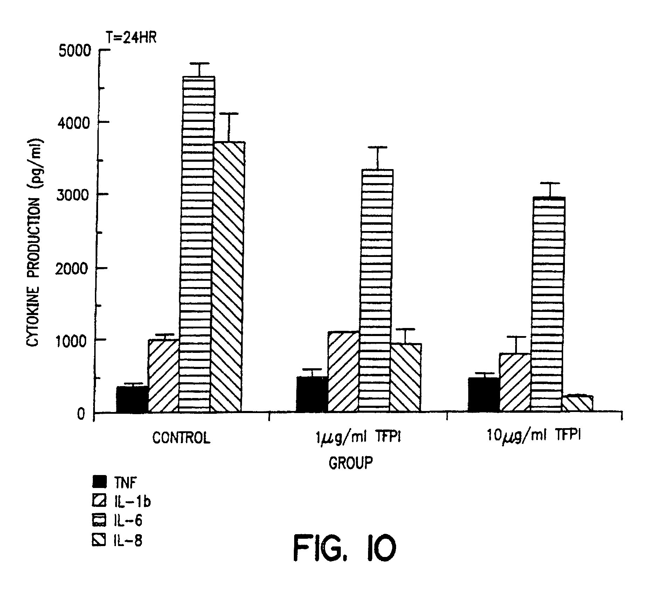

The described IL-8 response to the combination of coagulation activation/clotting + LPS is somewhat unique as the combination does not result in synergistic production of TNFα, IL-6 or IL-1β (Figure 10). Moreover, production of TNFα or IL-1β induced in low-hirudin+LPS cultures is not significantly inhibited by added TFPI concentrations to 10 µg/ml. While IL-6 production is slightly inhibited by TFPI, the IL-8 response is most significantly reduced by TFPI. The mechanism for the TFPI effect on induced IL-8 production in these cultures has not been determined. Without being bound to any particular theory, it may be that the ability of TFPI to inhibit coagulation activation by direct inhibition of factor Xa and the inhibition of factor VIIa/tissue factor in a Xa-dependent manner. However, it may be that TFPI has some ability to directly inhibit LPS activity.

### Example 3

The inhibitory effect of TFPI on the synthesis and release of neutrophil elastase, IL-8 and on plasmin may be used to assess the efficacy of treatment in patients with thrombosis disorders, patients with diseases associated with increased neutrophil elastase and in patients with diseases associated with increased IL-8. It is believed that the levels of neutrophil elastase, IL-8 and plasmin may be predictive of patient responsiveness to TFPI and prognosis. Patients who have received TFPI will have blood drawn and assayed for neutrophil elastase levels, for IL-8 levels, for plasmin activity or for any combination of these indicators. Levels for each of these indicators may be compared to an established historical baseline, as determined by sampling of populations of normal human volunteers. Alternatively, the level of neutrophil elastase, IL-8 or plasmin per patient may be followed over time prior to and after administration of TFPI. In the event that levels of the indicator or indicators tested have not decreased, additional dosing with TFPI may be required.

## Claims

1. Use of an agent in the manufacture of a medicament for treating a disease associated with increased synthesis and release of neutrophil elastase, said agent being capable of inhibiting (1) coagulation and (2) release of neutrophil elastase, wherein said agent is Tissue Factor Pathway Inhibitor (TFPI) or ala-TFPI, whereby one or more symptoms associated with said disease is reduced, and wherein the disease is selected from:
(a) severe acute pancreatitis;
(b) emphysema;
(c) multiple organ failure; and
(d) cystic fibrosis.

2. Use of an agent in the manufacture of a medicament for preventing a symptom of a disease associated with increased synthesis and release of neutrophil elastase, said agent being capable of inhibiting (1) coagulation and (2) release of neutrophil elastase, wherein said agent is Tissue Factor Pathway Inhibitor (TFPI) or ala-TFPI, whereby the risk of developing said symptom in the individual is reduced, and wherein the disease is selected from:
(a) severe acute pancreatitis;
(b) emphysema; and
(c) cystic fibrosis.

3. An *in vitro* method of measuring a patient's response to TFPI, comprising; determining the level of one or more indicators of Tissue Factor Pathway Inhibitor (TFPI) efficacy in a sample removed from the patient, said one or more indicators being selected from neutrophil elastase; IL-8; and plasmin, wherein a decreased level of said one or more indicators indicates a response of the patient to TFPI.

4. The method of claim 3 wherein the levels of neutrophil elastase; IL-8; and plasmin are determined.

5. The method of claim 3 or 4 wherein the patient has been diagnosed with one or more diseases associated with increased synthesis and release of neutrophil elastase selected from:
(a) severe acute pancreatitis;
(b) emphysema;
(c) rheumatoid arthritis;
(d) multiple organ failure;
(e) cystic fibrosis
(f) sepsis; and
(g) Adult Respiratory Distress Syndrome.

6. The method of claim 3 or 4 wherein the patient has been diagnosed with one or more diseases associated with increased synthesis and release of IL-8 selected from:
(a) Adult Respiratory Distress Syndrome;
(b) reperfusion injury;
(c) sepsis; and
(d) arthritis

7. The method of claim 3 or 4 wherein the patient has been diagnosed with a thrombosis related syndrome associated with increased synthesis and release of neutrophil elastase or increased synthesis and release of IL-8.

## Patentansprüche

1. Verwendung eines Agenses bei der Herstellung eines Medikaments zur Behandlung einer Krankheit, die mit erhöhter Synthese und Freisetzung von neutrophiler Elastase assoziiert ist, wobei das Agens fähig ist, (1) die Koagulation und (2) die Freisetzung neutrophiler Elastase zu inhibieren, das Mittel Gewebsthrombokinase-Bahn-Inhibitor (Tissue-Factor-Pathway-Inhibitor = TFPI) oder ala-TFPI ist, wodurch ein Symptom oder mehrere Symptome, die mit der Krankheit assoziiert sind, abgeschwächt wird/werden und wobei die Krankheit aus
(a) schwerer akuter Pancreatitis,
(b) Emphysem,
(c) mehrfachem Organversagen und
(d) zystischer Fibrose
ausgewählt ist.

2. Verwendung eines Agenses bei der Herstellung eines Medikaments zur Prävention eines Symptoms einer Krankheit, die mit erhöhter Synthese und Freisetzung von neutrophiler Elastase assoziiert ist, wobei das Mittel fähig ist, (1) die Koagulation und (2) die Freisetzung neutrophiler Elastase zu inhibieren, das Mittel Gewebsthrombokinase-Bahn-Inhibitor (Tissue-Factor-Pathway-Inhibitor = TFPI) oder ala-TFPI ist, wodurch die Gefahr einer Entwicklung des Symptoms bei dem Individuum reduziert wird und wobei die Krankheit aus:
(a) schwerer akuter Pancreatitis,
(b) Emphysem und
(c) zystischer Fibrose
ausgewählt ist.

3. In vitro-Verfahren zur Messung der Reaktion eines Patienten auf TFPI, umfassend Bestimmen des Levels eines oder mehrerer Indikatoren für die Gewebsthrombokinase-Bahn-Inhibitor (TFPI)-Wirksamkeit in einer Probe, die einem Patienten entnommen wurde, wobei der eine Indikator oder die mehreren Indikatoren aus neutrophiler Elastase, IL-8 und Plasmin ausgewählt wird/werden, wobei ein erniedrigter Level eines Indikators oder mehrerer Indikatoren eine Reaktion des Patienten auf TFPI anzeigt.

4. Verfahren nach Anspruch 3, wobei die Level an neutrophiler Elastase, IL-8 und Plasmin bestimmt werden.

5. Verfahren nach Anspruch 3 oder 4, wobei bei dem Patienten eine Krankheit oder mehrere Krankheiten diagnostiziert wurde(n), die mit erhöhter Synthese und Freisetzung neutrophiler Elastase assoziiert ist/sind, ausgewählt aus:
(a) schwerer akuter Pancreatitis,
(b) Emphysem,
(c) rheumatoider Arthritis,
(d) mehrfachem Organversagen,
(e) zystischer Fibrose,
(f) Sepsis und
(g) Atemnotsyndrom bei Erwachsenen.

6. Verfahren nach Anspruch 3 oder 4, wobei bei dem Patienten eine Krankheit diagnostiziert wurde oder mehrere Krankheiten diagnostiziert wurden, die mit erhöhter Synthese und Freisetzung von IL-8 assoziiert ist/sind, ausgewählt aus:
(a) Atemnotsyndrom bei Erwachsenen,
(b) Reperfusionschädigung,
(c) Sepsis und
(d) Arthritis.

7. Verfahren nach Anspruch 3 oder 4, wobei bei dem Patienten ein mit Thrombose in Verbindung stehendes Syndrom diagnostiziert wurde, das mit erhöhter Synthese und Freisetzung von neutrophiler Elastase oder erhöhter Synthese und Freisetzung von IL-8 assoziiert ist.

## Revendications

1. Utilisation d'un agent dans la fabrication d'un médicament pour le traitement d'une maladie associée à une synthèse et à une libération accrues d'élastase de neutrophile, ledit agent étant capable d'inhiber (1) la coagulation et (2) la libération d'élastase de neutrophile, dans laquelle ledit agent est l'inhibiteur de la voie du facteur tissulaire (TFPI) ou ala-TFPI, grâce à laquelle un ou plusieurs symptômes associés à ladite maladie sont réduits, et dans laquelle la maladie est choisie parmi :
(a) une pancréatite aiguë sévère ;
(b) un emphysème ;
(c) une défaillance polyviscérale ; et
(d) une fibrose kystique.

2. Utilisation d'un agent dans la fabrication d'un médicament pour prévenir un symptôme d'une maladie associée à une synthèse et à une libération accrues d'élastase de neutrophile, ledit agent étant capable d'inhiber (1) la coagulation et (2) la libération d'élastase de neutrophile, dans laquelle ledit agent est l'inhibiteur de la voie du facteur tissulaire (TFPI) ou ala-TFPI, grâce à laquelle le risque de développement dudit symptôme chez l'individu est réduit, et dans laquelle la maladie est choisie parmi :
(a) une pancréatite aiguë sévère ;
(b) un emphysème ; et
(c) une fibrose kystique.

3. Méthode *in vitro* de mesure d'une réponse d'un patient à TFPI, comprenant le fait de déterminer le niveau d'un ou plusieurs indicateurs de l'efficacité de l'inhibiteur de la voie du facteur tissulaire (TFPI) dans un échantillon prélevé sur le patient, lesdits un ou plusieurs indicateurs étant choisis parmi l'élastase de neutrophile ; l'IL-8 ; et la plasmine, dans laquelle un niveau réduit desdits un ou plusieurs indicateurs indique une réponse du patient à TFPI.

4. Méthode de la revendication 3, dans laquelle les niveaux d'élastase de neutrophile ; d'IL-8 ; et de plasmine sont déterminés.

5. Méthode de la revendication 3 ou 4, dans laquelle on a diagnostiqué chez le patient une ou plusieurs maladies associées à une synthèse et à une libération accrues d'élastase de neutrophile choisies parmi :
(a) une pancréatite aiguë sévère ;
(b) un emphysème ;
(c) une polyarthrite rhumatoïde
(d) une défaillance polyviscérale ;
(e) une fibrose kystique
(f) une septicémie ; et
(g) un syndrome de détresse respiratoire de l'adulte.

6. Méthode de la revendication 3 ou 4, dans laquelle on a diagnostiqué chez le patient une ou plusieurs maladies associées à une synthèse et à une libération accrues d'IL-8, choisies parmi :
(a) un syndrome de détresse respiratoire de l'adulte ;
(b) une lésion par reperfusion ;
(c) une septicémie ; et
(d) une arthrite

7. Méthode de la revendication 3 ou 4, dans laquelle on a diagnostiqué chez le patient un syndrome lié à une thrombose associée à une synthèse et à une libération accrues d'élastase de neutrophile ou à une synthèse et une libération accrues d'IL-8.
